# EUROPEAN PATENT APPLICATION

(11) **EP 4 733 395 A1**
(43) Date of publication of application: **29.04.2026**
(21) Application number: 24858228.0
(22) Date of filing: 05.08.2024
(51) Int. Cl.: C12N 9/22, C07K 19/00, C12N 15/55

(54) **CAS12J PROTEIN HAVING IMPROVED EDITING ACTIVITY AND USE THEREOF**

(30) Priority: 29.08.2023 CN 202311095245
(71) Applicant: Shandong Shunfeng Biotechnology Co., Ltd., Jinan, Shandong 250000 (CN)
(72) Inventor: DUAN, Zhiqiang, Jinan, Shandong 250000 (CN); CHEN, Ying, Jinan, Shandong 250000 (CN)
(74) Representative: Bayramoglu et al.
(86) International application number: PCT/CN2024/109759
(87) International publication number: WO 2025/044685

(57) **Abstract**

The present disclosure relates to the field of nucleic acid editing, and in particular to the technical field of clustered regularly interspaced short palindromic repeats (CRISPR). Specifically, the present disclosure provides a Cas12j mutant protein with an improved editing activity and a use thereof, and demonstrates promising application prospects.

## Description

The present application claims priority to Chinese Patent Application CN2023110952452 filed on August 29, 2023, which is incorporated herein by reference in its entirety.

### TECHNICAL FIELD

The present disclosure relates to the field of gene editing, and in particular to the technical field of clustered regularly interspaced short palindromic repeats (CRISPR). Specifically, the present disclosure relates to a Cas12j mutant protein with an enhanced activity and a use thereof.

### BACKGROUND TECHNOLOGY

The CRISPR/Cas technology is a widely used gene editing technology. In the CRISPR/Cas technology, the RNA guidance is used to specifically bind to a target sequence on the genome and cleave DNA to produce double-strand breaks, and the site-directed gene editing is conducted through biological non-homologous end joining or homologous recombination.

The CRISPR/Cas9 system is the most common type II CRISPR system. The CRISPR/Cas9 system recognizes the protospacer adjacent motif (PAM) of 3'-NGG and cleaves the target sequence to produce blunt-ended fragments. The CRISPR/Cas Type V system is a newly discovered CRISPR system. The CRISPR/Cas Type V system has a motif of 5'-TTN and can cleave a sticky end of a target sequence, such as Cpf1, C2c1, CasX, and CasY. However, the current different CRISPR/Cas systems have different advantages and disadvantages. For example, Cas9, C2c1, and CasX all require two RNAs for RNA guidance, while Cpf1 only requires one guide RNA (gRNA) and can be used for multiplex gene editing. CasX has a size of 980 amino acids, while common Cas9, C2c1, CasY, and Cpf1 usually have a size of about 1,300 amino acids. In addition, PAM sequences of Cas9, Cpf1, CasX, and CasY are all complex and diverse. C2c1 can only recognize the strict 5'-TTN, and thus a target site of C2c1 is more easily predicted than target sites of other systems, which reduces the potential off-target effect of C2c1.

Chinese patent CN111770992B discloses a Cas protein, Cas12j.19, and also discloses that this Cas protein enables the gene editing in eukaryotic cells. However, this Cas protein exhibits a low editing activity. To improve the editing efficiency of the Cas protein, the present application optimizes the Cas protein to improve the editing efficiency of the Cas protein in eukaryotic cells.

### CONTENT OF THE INVENTION

Through a large number of experiments and repeated explorations, the inventors of the present application conduct site-directed mutagenesis for Cas12j.19 (referred to as Cas12j19 in the present application) to improve an editing activity of the Cas protein and expand an application range of the Cas protein.

### Cas effector protein

In an aspect, the present disclosure provides a Cas mutant protein with an improved or enhanced activity. Corresponding to an amino acid sequence shown in SEQ ID No. 1, the Cas mutant protein includes a mutation of any one or more of a 3rd amino acid, a 4th amino acid, a 99th amino acid, a 154th amino acid, a 228th amino acid, a 231st amino acid, a 400th amino acid, a 411th amino acid, a 647th amino acid, a 649th amino acid, or a 658th amino acid relative to an amino acid sequence of a parental Cas protein.

In an embodiment, corresponding to the amino acid sequence shown in SEQ ID No. 1, the Cas mutant protein includes a mutation of any one or any more of the 3rd amino acid, the 4th amino acid, the 99th amino acid, the 154th amino acid, the 228th amino acid, the 231st amino acid, the 400th amino acid, the 411th amino acid, the 647th amino acid, the 649th amino acid, or the 658th amino acid relative to the amino acid sequence of the parental Cas protein, where the any more refers to any 2, any 3, any 4, any 5, any 6, any 7, any 8, any 9, any 10, or any 11.

In an embodiment, corresponding to the amino acid sequence shown in SEQ ID No. 1, the Cas mutant protein further includes a mutation of a 100th amino acid relative to the amino acid sequence of the parental Cas protein.

In an embodiment, corresponding to the amino acid sequence shown in SEQ ID No. 1, the Cas mutant protein includes the following amino acid mutations relative to the amino acid sequence of the parental Cas protein:
simultaneous mutations of 100th and 3rd amino acids; or
simultaneous mutations of 100th and 99th amino acids; or
simultaneous mutations of 100th and 154th amino acids; or
simultaneous mutations of 100th and 231st amino acids; or
simultaneous mutations of 100th and 400th amino acids; or
simultaneous mutations of 100th and 411th amino acids; or
simultaneous mutations of 100th and 647th amino acids; or
simultaneous mutations of 100th and 649th amino acids; or
simultaneous mutations of 100th and 658th amino acids; or
simultaneous mutations of 100th, 400th, and 763rd amino acids; or
simultaneous mutations of 100th, 400th, 763rd, and 45th amino acids.

In an embodiment, the 3rd amino acid is mutated to an amino acid other than S, for example, A, V, G, L, D, F, W, Y, N, E, Q, K, M, T, C, P, H, R, or I, and preferably R.

In an embodiment, the 4th amino acid is mutated to an amino acid other than Y, for example, A, V, G, L, D, F, W, S, N, E, Q, K, M, T, C, P, H, R, or I, and preferably R.

In an embodiment, the 99th amino acid is mutated to an amino acid other than L, for example, A, V, G, Y, D, F, W, S, N, E, Q, K, M, T, C, P, H, R, or I, and preferably R.

In an embodiment, the 154th or 231st amino acid is mutated to an amino acid other than A, for example, L, V, G, Y, D, F, W, S, N, E, Q, K, M, T, C, P, H, R, or I, and preferably R.

In an embodiment, the 228th or 400th amino acid is mutated to an amino acid other than S, for example, A, V, G, Y, D, F, W, L, N, E, Q, K, M, T, C, P, H, R, or I, and preferably R.

In an embodiment, the 411th or 647th amino acid is mutated to an amino acid other than T, for example, A, V, G, L, D, F, W, Y, N, E, Q, K, M, S, C, P, H, R, or I, and preferably R.

In an embodiment, the 649th or 658th amino acid is mutated to an amino acid other than D, for example, A, V, G, L, S, F, W, Y, N, E, Q, K, M, T, C, P, H, R, or I, and preferably R.

In an embodiment, the 100th amino acid is mutated to an amino acid other than E, for example, A, V, G, L, S, F, W, Y, N, D, Q, K, M, T, C, P, H, R, or I, and preferably K.

In an embodiment, the 763rd amino acid is mutated to an amino acid other than L, for example, A, V, G, Y, D, F, W, S, N, E, Q, K, M, T, C, P, H, R, or I, and preferably R.

In an embodiment, the 45th amino acid is mutated to an amino acid other than S, for example, A, V, G, Y, D, F, W, L, N, E, Q, K, M, T, C, P, H, R, or I, and preferably T.

In an embodiment, the amino acid sequence of the parental Cas protein has a sequence identity of at least 70%, at least 80%, at least 85%, at least 90%, at least 91%, at least 92%, at least 93%, at least 94%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, or at least 99.9% with SEQ ID No. 1.

In an embodiment, the parental Cas protein is a Cas protein of a Cas12 family and is preferably a Cas protein of a Cas12j family, such as Cas12j.3 to Cas12j.22 disclosed in CN111770992B. In a preferred embodiment, the parental Cas protein is Cas12j19. An amino acid sequence of the Cas12j19 has a sequence identity of at least 70%, at least 80%, at least 85%, at least 90%, at least 91%, at least 92%, at least 93%, at least 94%, at least 95%, at least 96%, at least 97%, at least 98%, at least 99%, at least 99.1%, at least 99.2%, at least 99.3%, at least 99.4%, at least 99.5%, at least 99.6%, at least 99.7%, at least 99.8%, at least 99.9%, or 100% with SEQ ID No. 1.

Cas proteins or Cas12j proteins from various organisms can be adopted as the parental Cas protein. In some embodiments, the parental Cas protein or Cas12j protein has a nuclease activity. In some embodiments, the parental Cas protein is a nuclease, which can cleave two strands of a target double-stranded nucleic acid (such as double-stranded DNA). In some embodiments, the parental Cas protein is a nickase, which can cleave a single strand of a target double-stranded nucleic acid (such as double-stranded DNA).

In some embodiments, the parental Cas protein is a natural wild-type (WT) Cas protein. In other embodiments, the parental Cas protein is an engineered Cas protein.

It is clear to those skilled in the art that a structure of a protein can be changed without adversely affecting the activity and functionality of the protein. For example, one or more conservative amino acid substitutions can be introduced into an amino acid sequence of a protein without adversely affecting the activity and/or three-dimensional (3D) structure of the protein molecule. Those skilled in the art are aware of examples and implementations of the conservative amino acid substitutions. Specifically, an amino acid residue can be substituted by another amino acid residue that belongs to the same group as the amino acid residue to be substituted. That is, a nonpolar amino acid residue can be substituted by another nonpolar amino acid residue, an uncharged polar amino acid residue can be substituted by another uncharged polar amino acid residue, a basic amino acid residue can be substituted by another basic amino acid residue, and an acidic amino acid residue can be substituted by another acidic amino acid residue. Such substituted amino acid residues may be or may not be encoded by genetic codes. As long as a substitution does not result in a loss of a biological activity of a protein, a conservative substitution in which an amino acid is substituted by another amino acid belonging to the same group falls within the scope of the present disclosure. Therefore, an amino acid sequence of the protein of the present disclosure may include one or more conservative substitutions, and these conservative substitutions may be preferably generated according to Table 1. In addition, the present disclosure covers proteins with one or more other non-conservative substitutions, as long as the non-conservative substitutions do not significantly affect the desired function and biological activity of the protein of the present disclosure.

The conservative amino acid substitutions can be generated at one or more predicted non-essential amino acid residues. "Non-essential" amino acid residues are amino acid residues that can be changed (deleted or substituted) without changing the biological activity, while "essential" amino acid residues are required for the biological activity. "A conservative amino acid substitution" refers to a substitution in which an amino acid residue is substituted by an amino acid residue with a similar side chain. An amino acid substitution can be conducted in a non-conservative region of the Cas mutant protein described above. Generally, such a substitution is not conducted to a conservative amino acid residue or an amino acid residue located within a conservative motif because such a residue is necessary for the activity of a protein. However, those skilled in the art should understand that a functional variant may have few conservative or non-conservative variations in conservative regions.

**Table 1**

| Initial residue | Representative substitution | Preferred substitution |
|---|---|---|
| Ala (A) | Val; Leu; Ile | Val |
| Arg (R) | Lys; Gln; Asn | Lys |
| Asn (N) | Gln; His; Lys; Arg | Gln |
| Asp (D) | Glu | Glu |
| Cys (C) | Ser | Ser |
| Gln (Q) | Asn | Asn |
| Glu (E) | Asp | Asp |
| Gly (G) | Pro; Ala | Ala |
| His (H) | Asn; Gln; Lys; Arg | Arg |
| Ile (I) | Leu; Val; Met; Ala; Phe | Leu |
| Leu (L) | Ile; Val; Met; Ala; Phe | Ile |
| Lys (K) | Arg; Gln; Asn | Arg |
| Met (M) | Leu; Phe; Ile | Leu |
| Phe (F) | Leu; Val; Ile; Ala; Tyr | Leu |
| Pro (P) | Ala | Ala |
| Ser (S) | Thr | Thr |
| Thr (T) | Ser | Ser |
| Trp (W) | Tyr; Phe | Tyr |
| Tyr (Y) | Trp; Phe; Thr; Ser | Phe |
| Val (V) | Ile; Leu; Met; Phe; Ala | Leu |

It is well known in the art that one or more amino acid residues can be changed (substituted, deleted, truncated, or inserted) at an N-terminus and/or a C-terminus of a protein with the functional activity of the protein still retained. Therefore, a protein produced by changing one or more amino acid residues at an N-terminus and/or a C-terminus of the Cas protein while retaining a desired functional activity also falls within the scope of the present disclosure. The change may include a change introduced by a modem molecular method such as polymerase chain reaction (PCR). The modem molecular method includes PCR amplification that alters or extends a protein coding sequence by introducing an amino acid coding sequence into an oligonucleotide used for the PCR amplification.

It should be recognized that a protein can be altered in various ways, including amino acid substitution, deletion, truncation, and insertion, and methods for such operations are generally known in the art. For example, amino acid sequence variants of the protein can be prepared through a mutation of DNA. It can also be completed through other mutagenesis forms and/or through directed evolution. For example, known mutagenesis, recombination, and/or shuffling methods can be used in combination with a related screening method to achieve the substitution, deletion, and/or insertion of one or more amino acids.

Those skilled in the art can understand that these small amino acid changes in the Cas protein of the present disclosure can naturally occur (for example, natural mutations) or can be induced (for example, using r-DNA technology) without affecting a function or an activity of the protein. If these mutations occur in a catalytic domain, an active site, or another functional domain of the protein, the properties of the polypeptide may be changed, but an activity of the polypeptide may be retained. If the existing mutations are not close to a catalytic domain, an active site, or another functional domain, it can be expected that there is a small impact.

Those skilled in the art can identify the essential amino acids of the Cas mutant protein of the present disclosure according to a method known in the art, such as site-directed mutagenesis or protein evolution or bioinformatics analysis. The catalytic domain, active site, or another functional domain of the protein can also be determined through the physical analysis of the structure, for example, it can be determined by a technique such as nuclear magnetic resonance (NMR), crystallography, electron diffraction, or photoaffinity labeling in combination with a putative amino acid mutation at a key site.

In the present disclosure, an amino acid residue can be represented by a single letter or three letters, such as: alanine (Ala, A), valine (Val, V), glycine (Gly, G), leucine (Leu, L), glutamine (Gln, Q), phenylalanine (Phe, F), tryptophan (Trp, W), tyrosine (Tyr, Y), aspartic acid (Asp, D), asparagine (Asn, N), glutamic acid (Glu, E), lysine (Lys, K), methionine (Met, M), serine (Ser, S), threonine (Thr, T), cysteine (Cys, C), proline (Pro, P), isoleucine (Ile, I), histidine (His, H), and arginine (Arg, R).

The term "AxxB" means that an amino acid A at position xx is mutated to an amino acid B, that is, the amino acid A at the position xx from an N-terminus is mutated to the amino acid B, unless otherwise specified. For example, E100K indicates that E at position 100 is mutated to K. When there are mutations simultaneously at a plurality of amino acid sites, the mutations can be expressed in a form such as E100K-S3R. For example, E100K-S3R means that E at position 100 is mutated to K and S at position 3 is mutated to R.

A specific amino acid position (number) in the protein of the present disclosure is determined by aligning an amino acid sequence of the target protein with SEQ ID No. 1 using a standard sequence alignment tool. For example, the Smith-Waterman algorithm or the CLUSTALW2 algorithm is used to align two sequences, and the sequences are considered aligned when an alignment score is the highest. The alignment score can be calculated according to the method described in Wilbur, W. J. and Lipman, D. J. (1983) Rapid similarity searches of nucleic acid and protein data banks. Proc. Natl. Acad. Sci. USA, 80: 726-730. In the ClustalW2(1.82) algorithm, default parameters are preferably adopted: protein gap opening penalty = 10.0; protein gap extension penalty = 0.2; protein matrix = Gonnet; protein/DNA end gap = -1; and protein/DNAGAPDIST = 4. The AlignX program (a part of the vectorNTI group) may preferably be used to align an amino acid sequence of the protein of the present disclosure with SEQ ID No. 1 under default parameters suitable for multiple alignment (gap opening penalty: 10og; and gap extension penalty: 0.05) to determine a position of a specific amino acid in the protein.

In an embodiment, the Cas mutant protein is selected from any one of the following I to III:
I. a Cas mutant protein produced through a mutation of any one or more of a 3rd amino acid, a 4th amino acid, a 99th amino acid, a 154th amino acid, a 228th amino acid, a 231st amino acid, a 400th amino acid, a 411th amino acid, a 647th amino acid, a 649th amino acid, or a 658th amino acid in the amino acid sequence shown in SEQ ID No. 1;
II. a Cas mutant protein that has the same mutation site as the Cas mutant protein defined in the I and has a sequence identity of at least 80%, at least 85%, at least 90%, at least 91%, at least 92%, at least 93%, at least 94%, at least 95%, at least 96%, at least 97%, at least 98%, or at least 99% with the Cas mutant protein defined in the I; and
III. a sequence that has the same mutation site as the Cas mutant protein defined in the I and includes substitution, deletion, or addition of one or more amino acids relative to the Cas mutant protein defined in the I, where the one or more amino acids include 1, 2, 3, 4, 5, 6, 7, 8, 9, or 10 amino acids.

In an embodiment, the Cas mutant protein is selected from any one of the following I to III:
I. a Cas mutant protein produced through a mutation of any one or more of a 3rd amino acid, a 4th amino acid, a 99th amino acid, a 154th amino acid, a 228th amino acid, a 231st amino acid, a 400th amino acid, a 411th amino acid, a 647th amino acid, a 649th amino acid, or a 658th amino acid in the amino acid sequence shown in SEQ ID No. 1, where the Cas mutant protein further includes a mutation of any one or more of a 100th amino acid, a 763rd amino acid, or a 43rd amino acid relative to the amino acid sequence shown in SEQ ID No. 1;
II. a Cas mutant protein that has the same mutation site as the Cas mutant protein defined in the I and has a sequence identity of at least 80%, at least 85%, at least 90%, at least 91%, at least 92%, at least 93%, at least 94%, at least 95%, at least 96%, at least 97%, at least 98%, or at least 99% with the Cas mutant protein defined in the I; and
III. a sequence that has the same mutation site as the Cas mutant protein defined in the I and includes substitution, deletion, or addition of one or more amino acids relative to the Cas mutant protein defined in the I, where the one or more amino acids include 1, 2, 3, 4, 5, 6, 7, 8, 9, or 10 amino acids.

In an embodiment, the Cas mutant protein is selected from any one of the following I to III:
I. a Cas mutant protein produced through a mutation of any one or more of a 3rd amino acid, a 4th amino acid, a 99th amino acid, a 154th amino acid, a 228th amino acid, a 231st amino acid, a 400th amino acid, a 411th amino acid, a 647th amino acid, a 649th amino acid, or a 658th amino acid in the amino acid sequence shown in SEQ ID No. 1, where the Cas mutant protein includes a 100th amino acid of K relative to the amino acid sequence shown in SEQ ID No. 1;
II. a Cas mutant protein that has the same mutation site as the Cas mutant protein defined in the I and has a sequence identity of at least 80%, at least 85%, at least 90%, at least 91%, at least 92%, at least 93%, at least 94%, at least 95%, at least 96%, at least 97%, at least 98%, or at least 99% with the Cas mutant protein defined in the I; and
III. a sequence that has the same mutation site as the Cas mutant protein defined in the I and includes substitution, deletion, or addition of one or more amino acids relative to the Cas mutant protein defined in the I, where the one or more amino acids include 1, 2, 3, 4, 5, 6, 7, 8, 9, or 10 amino acids. A biological function of the Cas protein includes, but is not limited to, an activity to bind to gRNA, an endonuclease activity, and an activity to bind to a specific site of a target sequence and cleave the target sequence under the guidance of gRNA, including, but not limited to, Cis cleavage activity and Trans cleavage activity.

In the present disclosure, the "Cas mutant protein" can also be referred to as a mutant Cas protein or a Cas protein variant.

The present disclosure also provides a fusion protein including the Cas mutant protein and other modification moieties.

In an embodiment, the modification moieties are selected from other proteins or polypeptides, detectable markers, or any combination thereof.

In an embodiment, the modification moieties are selected from an epitope tag, a reporter gene sequence, a nuclear localization signal (NLS) sequence, a targeting moiety, a transcriptional activation domain (such as VP64), a transcriptional repression domain (such as KRAB or SID domain), a nuclease domain (such as Fok1), and a domain with an activity selected from the following: a nucleotide deaminase activity, an adenosine deaminase activity, a cytidine deaminase activity, a methylase activity, a demethylase activity, a transcription activation activity, a transcription repression activity, a transcription release factor activity, a histone modification activity, a nuclease activity, a single-stranded RNA cleavage activity, a double-stranded RNA cleavage activity, a single-stranded DNA cleavage activity, a double-stranded DNA cleavage activity, and a nucleic acid binding activity; and any combination thereof. The NLS sequence is well known to those skilled in the art, and examples thereof include, but are not limited to, SV40 large T antigen, EGL-13, c-Myc, and TUS protein.

In an embodiment, the NLS sequence is located at, close to, or proximate to a terminus (such as an N-terminus, a C-terminus, or both termini) of the Cas protein of the present disclosure.

The epitope tag is well known to those skilled in the art, including but not limited to His, V5, FLAG, HA, Myc, VSV-G, and Trx. Those skilled in the art can select other suitable epitope tags (such as purification, detection, or tracing tags).

The reporter gene sequence is well known to those skilled in the art, and examples thereof include, but are not limited to, GST, HRP, CAT, GFP, HcRed, DsRed, CFP, YFP, and BFP.

In an embodiment, the fusion protein of the present disclosure includes a domain capable of binding to a DNA molecule or an intracellular molecule, such as a maltose-binding protein (MBP), a DNA binding domain (DBD) of Lex A, and DBD of GAL4.

In an embodiment, the fusion protein of the present disclosure includes a detectable marker, such as a fluorescent dye, such as fluorescein isothiocyanate (FITC) or 4',6-diamidino-2-phenylindole (DAPI).

In an embodiment, the Cas protein of the present disclosure may be optionally coupled to, conjugated with, or fused to the modification moiety through a linker.

In an embodiment, the modification moiety is directly linked to the N-terminus or C-terminus of the Cas protein of the present disclosure.

In an embodiment, the modification moiety is linked to the N-terminus or C-terminus of the Cas protein of the present disclosure through a linker. Such a linker is well known in the art, and examples thereof include, but are not limited to, a linker with one or more (such as 1, 2, 3, 4, or 5) amino acids (such as Glu or Ser) or amino acid derivatives (such as Ahx, β-Ala, GABA, or Ava), a polyethylene glycol (PEG) linker, etc.

A production method of the Cas protein, the protein derivative, or the fusion protein of the present disclosure is not limited. For example, the Cas protein, the protein derivative, or the fusion protein can be produced by a genetic engineering method (recombinant technology), or can be produced by a chemical synthesis method.

### Nucleic acid of the Cas protein

In another aspect, the present disclosure provides an isolated polynucleotide, including:
(a) a polynucleotide sequence encoding the Cas mutant protein or the fusion protein of the present disclosure, or
a polynucleotide complementary to the polynucleotide in (a).

In an embodiment, the polynucleotide sequence is codon-optimized for expression in a prokaryotic cell. In an embodiment, the polynucleotide sequence is codon-optimized for expression in a eukaryotic cell.

In an embodiment, the cell is an animal cell, such as a mammalian cell.

In an embodiment, the cell is a human cell.

In an embodiment, the cell is a plant cell, such as a cell possessed by a cultivated plant (such as cassava, corn, sorghum, wheat, or rice), an alga, a tree, or a vegetable.

In an embodiment, the polynucleotide is preferably single-stranded or double-stranded.

### gRNA

In another aspect, the present disclosure provides a gRNA, including a first segment and a second segment. The first segment is also called "framework region", "protein binding segment", "protein binding sequence", or "direct repeat". The second segment is also called "nucleic acid-targeted targeting sequence", "nucleic acid-targeted targeting segment", or "target sequence-targeted guide sequence".

The first segment of the gRNA can interact with the Cas protein of the present disclosure, such that the Cas protein and the gRNA can produce a complex.

In a preferred embodiment, the first segment is the direct repeat above.

The nucleic acid-targeted targeting sequence or the nucleic acid-targeted targeting segment of the present disclosure includes a nucleotide sequence complementary to a sequence in a target nucleic acid. In other words, the nucleic acid-targeted targeting sequence or the nucleic acid-targeted targeting segment of the present disclosure can interact with the target nucleic acid in a sequence-specific manner through hybridization (namely, base pairing). Therefore, the nucleic acid-targeted targeting sequence or the nucleic acid-targeted targeting segment can be changed, or can be modified to hybridize with any desired sequence in the target nucleic acid. The nucleic acid is selected from DNA or RNA.

The nucleic acid-targeted targeting sequence or the nucleic acid-targeted targeting segment can have a complementarity percentage of at least 60% (such as at least 65%, at least 70%, at least 75%, at least 80%, at least 85%, at least 90%, at least 95%, at least 97%, at least 98%, at least 99%, or 100%) with a target sequence of the target nucleic acid.

The "framework region", "protein binding segment", "protein binding sequence", or "direct repeat" of the gRNA of the present disclosure can interact with the CRISPR protein (or the Cas protein). The gRNA of the present disclosure guides the Cas protein interacting with the gRNA to a specific nucleotide sequence in the target nucleic acid under the action of the nucleic acid-targeted targeting sequence.

Preferably, the gRNA includes a first segment and a second segment in a direction from 5'-terminus to 3'-terminus.

In the present disclosure, the second segment can also be understood as a guide sequence to hybridize with a target sequence.

The gRNA of the present disclosure can produce a complex with the Cas protein.

### Vector

The present disclosure also provides a vector, including the Cas mutant protein, the isolated nucleic acid, or the polynucleotide described above. Preferably, the vector also includes a regulatory element operably linked to the Cas mutant protein, the isolated nucleic acid, or the polynucleotide.

In an embodiment, the regulatory element is one or more of an enhancer, a transposon, a promoter, a terminator, a leader sequence, a polyadenylate sequence, and a marker gene.

In an embodiment, the vector includes a cloning vector, an expression vector, a shuttle vector, and an integration vector.

In some embodiments, a vector included in the system is a viral vector (such as a retroviral vector, a lentiviral vector, an adenoviral vector, an adeno-associated virus vector, or a herpes simplex virus vector), and may also be a plasmid, a virus, a cosmid, a phage, etc., which are well known to those skilled in the art.

### CRISPR system

The present disclosure provides an engineered non-natural vector system or a CRISPR-Cas system, which includes the Cas mutant protein or a nucleic acid sequence encoding the Cas mutant protein, and a nucleic acid encoding one or more gRNAs.

In an embodiment, the nucleic acid sequence encoding the Cas mutant protein and the nucleic acid encoding one or more gRNAs are artificially synthesized.

In an embodiment, the nucleic acid sequence encoding the Cas mutant protein and the nucleic acid encoding one or more gRNAs do not co-exist naturally.

The one or more gRNAs target one or more target sequences in a cell. The one or more target sequences hybridize with a genomic locus of a DNA molecule encoding one or more gene products, and guide the Cas protein to reach the genomic locus of the DNA molecule encoding the one or more gene products. After the Cas protein reaches a position of a target sequence, the target sequence is modified, edited, or cleaved, such that the expression of the one or more gene products is changed or modified.

The cell of the present disclosure includes one or more of an animal cell, a plant cell, and a microorganism.

In some embodiments, the Cas protein is codon-optimized for expression in a cell.

In some embodiments, the Cas protein guides the cleavage of one or two strands at the position of the target sequence.

The present disclosure also provides an engineered non-natural vector system, including one or more vectors. The one or more vectors include:
a) a first regulatory element operably linked to the gRNA and
b) a second regulatory element operably linked to the Cas protein.

The components (a) and (b) are located on a same vector or different vectors of the system.

The first and second regulatory elements each include a promoter (such as a constitutive promoter or an inducible promoter), an enhancer (such as a 35S promoter or a 35S enhanced promoter), an internal ribosome entry site (IRES), and other expression control elements (such as a transcriptional termination signal, such as a polyadenylation signal and a poly-U sequence).

In some embodiments, a vector in the system is a viral vector (such as a retroviral vector, a lentiviral vector, an adenoviral vector, an adeno-associated virus vector, and a herpes simplex virus vector), and may also be a plasmid, a virus, a cosmid, a phage, etc., which are well known to those skilled in the art.

In some embodiments, the system provided herein is in a delivery system. In some embodiments, the delivery system is a nanoparticle, a liposome, an exosome, a microvesicle, and a gene gun.

In an embodiment, the target sequence is a DNA or RNA sequence derived from a prokaryotic cell or a eukaryotic cell. In an embodiment, the target sequence is a non-natural DNA or RNA sequence.

In an embodiment, the target sequence is present in a cell. In an embodiment, the target sequence is present in the nucleus or cytoplasm (such as an organelle). In an embodiment, the cell is a eukaryotic cell. In other embodiments, the cell is a prokaryotic cell.

In an embodiment, the Cas protein is linked to one or more NLS sequences. In an embodiment, the fusion protein includes one or more NLS sequences. In an embodiment, the NLS sequence is linked to an N-terminus or a C-terminus of the protein. In an embodiment, the NLS sequence is fused to an N-terminus or a C-terminus of the protein.

In another aspect, the present disclosure relates to an engineered CRISPR system, including the Cas protein and one or more gRNAs. The gRNAs include a direct repeat and a spacer capable of hybridizing with a target nucleic acid. The Cas protein can bind to the gRNAs and target the target nucleic acid sequence complementary to the spacer.

### Protein-nucleic acid complex/composition

In another aspect, the present disclosure provides a complex or a composition, including:
(i) a protein component selected from the Cas protein, a derivatized protein, or the fusion protein and any combination thereof; and
(ii) a nucleic acid component including: (a) a guide sequence capable of hybridizing with a target sequence and (b) a direct repeat capable of binding to the Cas protein of the present disclosure.

The protein component binds to the nucleic acid component to produce a complex.

In an embodiment, the nucleic acid component is a gRNA in the CRISPR-Cas system.

In an embodiment, the complex or the composition is non-natural or modified. In an embodiment, at least one component in the complex or the composition is non-natural or modified. In an embodiment, the first component is non-natural or modified; and/or, the second component is non-natural or modified.

### Activated CRISPR complex

In another aspect, the present disclosure also provides an activated CRISPR complex, including: (1) a protein component selected from the Cas protein, the derivatized protein, or the fusion protein of the present disclosure and any combination thereof; (2) gRNA including: (a) a guide sequence capable of hybridizing with a target sequence and (b) a direct repeat capable of binding to the Cas protein of the present disclosure; and (3) a target sequence binding to the gRNA. Preferably, the binding refers to the binding to a target nucleic acid through a nucleic acid-targeted targeting sequence on the gRNA.

The term "activated CRISPR complex", "activated complex", or "ternary complex" used herein refers to a complex produced after the Cas protein and gRNA in the CRISPR system bind to a target nucleic acid or are modified.

The Cas protein and the gRNA of the present disclosure can produce a binary complex. The binary complex is activated when binding to a nucleic acid substrate to produce an activated CRISPR complex in which the nucleic acid substrate is complementary to a spacer (or called a guide sequence to hybridize with the target nucleic acid) in the gRNA. In some embodiments, the spacer of the gRNA exactly matches the target substrate. In other embodiments, the spacer of the gRNA matches a portion (continuous or discontinuous) of the target substrate.

In a preferred embodiment, the activated CRISPR complex can exhibit a side-branch nuclease cleavage activity. The side-branch nuclease cleavage activity refers to a non-specific cleavage activity or a disordered cleavage activity, also called a trans cleavage activity in the art, of the activated CRISPR complex for a single-stranded nucleic acid.

### Delivery and delivery composition

The Cas protein, gRNA, fusion protein, nucleic acid, vector, system, complex, and composition of the present disclosure can be delivered by any method known in the art. Such a method includes, but is not limited to, electroporation, lipofection, nucleofection, microinjection, sonoporation, gene gun, calcium phosphate-mediated transfection, cationic lipid transfection, lipofectin transfection, dendritic transfection, heat-shock transfection, nucleofection, magnetofection, lipofection, puncture transfection, optical transfection, reagent-enhanced nucleic acid intake, and delivery through a liposome, an immunoliposome, a viral particle, an artificial virus, etc.

Therefore, in another aspect, the present disclosure provides a delivery composition, including a delivery vehicle and one or more selected from the following: the Cas protein, the fusion protein, the nucleic acid, the vector, the system, the complex, and the composition of the present disclosure.

In an embodiment, the delivery vehicle is a particle.

In an embodiment, the delivery vehicle is selected from a lipid particle, a sugar particle, a metal particle, a protein particle, a liposome, an exosome, a microvesicle, a gene gun, or a viral vector (such as a replication-defective retrovirus, a lentivirus, an adenovirus, or an adeno-associated virus).

### Host cell

The present disclosure also relates to a cell or cell line or a progeny thereof *in vitro* or *in vivo.* The cell or cell line or the progeny thereof includes the Cas protein, the fusion protein, the nucleic acid, the protein-nucleic acid complex, the activated CRISPR complex, the vector, or the delivery composition of the present disclosure.

In some embodiments, the cell is a prokaryotic cell.

In some embodiments, the cell is a eukaryotic cell. In some embodiments, the cell is a mammalian cell. In some embodiments, the cell is a human cell. In some embodiments, the cell is a non-human mammalian cell, such as a cell of a non-human primate, cow, sheep, pig, dog, monkey, rabbit, or a rodent (such as a rat or a mouse). In some embodiments, the cell is a non-mammalian eukaryotic cell, such as a cell of a poultry bird (such as chicken), fish, or crustacea (such as clam or shrimp). In some embodiments, the cell is a plant cell, such as a cell possessed by a monocotyledonous plant or a dicotyledonous plant or a cell possessed by a cultivated plant or a food crop such as cassava, corn, sorghum, soybean, wheat, oats, or rice, such as an alga, a tree or a production plant, a fruit, or a vegetable (for example, a tree such as a citrus tree or a nut tree; or a nightshade, cotton, tobacco, tomato, grape, coffee, cocoa, etc.).

In some embodiments, the cell is a stem cell or a stem cell line.

In some cases, the host cell of the present disclosure includes a modification for a gene or a genome, and the modification is not present in a WT of the host cell.

### Gene editing method and use

The Cas mutant protein, the nucleic acid, the composition, the CIRSPR/Cas system, the vector system, the delivery composition or the activated CRISPR complex, or the host cell of the present disclosure can be used in any one or more of the following: targeting and/or editing a target nucleic acid; cleaving a double-stranded DNA, a single-stranded DNA, or a single-stranded RNA; non-specifically cleaving and/or degrading a side-branch nucleic acid; non-specifically cleaving a single-stranded nucleic acid; nucleic acid detection; detecting a nucleic acid in a target sample; specifically editing a double-stranded nucleic acid; base-editing a double-stranded nucleic acid; and base-editing a single-stranded nucleic acid. In other embodiments, the products of the present disclosure can also be used to prepare a reagent or a kit for any one or more of the above purposes.

The present disclosure also provides a use of the Cas protein, the nucleic acid, the composition, the CIRSPR/Cas system, the vector system, the delivery composition, or the activated CRISPR complex in gene editing, gene targeting, or gene cleaving; or a use thereof in preparation of a reagent or a kit for gene editing, gene targeting, or gene cleaving.

In an embodiment, the gene editing, the gene targeting, or the gene cleaving refers to gene editing, gene targeting, or gene cleaving inside and/or outside a cell.

The present disclosure also provides a method for editing a target nucleic acid, targeting a target nucleic acid, or cleaving a target nucleic acid, including: making the target nucleic acid in contact with the Cas protein, the nucleic acid, the composition, the CIRSPR/Cas system, the vector system, the delivery composition, or the activated CRISPR complex. In an embodiment, the method includes: editing, targeting, or cleaving the target nucleic acid inside or outside a cell.

The gene editing or the editing a target nucleic acid includes modifying a gene, knocking out a gene, changing the expression of a gene product, repairing a mutation, and/or inserting a polynucleotide or a gene mutation.

The editing can be conducted in a prokaryotic cell and/or a eukaryotic cell.

In another aspect, the present disclosure also provides a use of the Cas protein, the nucleic acid, the composition, the CIRSPR/Cas system, the vector system, the delivery composition, or the activated CRISPR complex in nucleic acid detection or in preparation of a reagent or a kit for nucleic acid detection.

In another aspect, the present disclosure also provides a method for cleaving a single-stranded nucleic acid, including: making a nucleic acid group in contact with the Cas protein and the gRNA described above. The nucleic acid group includes a target nucleic acid and a plurality of non-target single-stranded nucleic acids, and the Cas protein cleaves the plurality of non-target single-stranded nucleic acids.

The gRNA can bind to the Cas protein.

The gRNA can target the target nucleic acid.

The contact can be conducted inside a cell *in vitro* or *in vivo.*

Preferably, the cleavage of the single-stranded nucleic acid refers to non-specific cleavage.

In another aspect, the present disclosure also provides a use of the Cas protein, the nucleic acid, the composition, the CIRSPR/Cas system, the vector system, the delivery composition, or the activated CRISPR complex in non-specific cleavage of a single-stranded nucleic acid or in preparation of a reagent or kit for non-specific cleavage of a single-stranded nucleic acid.

In another aspect, the present disclosure also provides a kit for gene editing, gene targeting, or gene cleaving. The kit includes the Cas protein, the gRNA, the nucleic acid, the composition, the CIRSPR/Cas system, the vector system, the delivery composition, the activated CRISPR complex, or the host cell.

In another aspect, the present disclosure also provides a kit for detecting a target nucleic acid in a sample. The kit includes: (a) the Cas protein or a nucleic acid encoding the Cas protein; (b) the gRNA, or a nucleic acid encoding the gRNA, or a precursor RNA including the gRNA, or a nucleic acid encoding the precursor RNA; and (c) a single-stranded nucleic acid detector that does not hybridize with the gRNA.

It is known in the art that the precursor RNA can be cleaved or processed into the above-mentioned mature gRNA.

In another aspect, the present disclosure provides a use of the Cas protein, the nucleic acid, the composition, the CIRSPR/Cas system, the vector system, the delivery composition, the activated CRISPR complex, or the host cell in preparation of a formulation or a kit. The formulation or the kit is provided for:
(i) gene or genome editing;
(ii) target nucleic acid detection and/or diagnosis;
(iii) editing a target sequence in a target gene locus to modify an organism or a non-human organism;
(iv) disease treatment; and
(iv) targeting a target gene.

Preferably, the gene or genome editing is conducted inside or outside a cell.

Preferably, the target nucleic acid detection and/or diagnosis is conducted *in vitro.*

Preferably, the disease treatment refers to a treatment of a disease caused by a defect of a target sequence in a target gene locus.

In another aspect, the present disclosure provides a method for detecting a target nucleic acid in a sample, including: making the sample in contact with the Cas protein, gRNA, and a single-stranded nucleic acid detector; and detecting a detectable signal generated due to cleavage of the single-stranded nucleic acid detector by the Cas protein to detect the target nucleic acid. The gRNA includes a region to bind to the Cas protein and a guide sequence to hybridize with a target nucleic acid, and the single-stranded nucleic acid detector does not hybridize with the gRNA.

### Method for specifically modifying a target nucleic acid

In another aspect, the present disclosure also provides a method for specifically modifying a target nucleic acid, including: making the target nucleic acid in contact with the Cas protein, the nucleic acid, the composition, the CIRSPR/Cas system, the vector system, the delivery composition, or the activated CRISPR complex.

This specific modification may occur *in vivo* or *in vitro.*

This specific modification may occur inside or outside a cell.

In some cases, the cell is selected from a prokaryotic cell or a eukaryotic cell, such as an animal cell, a plant cell, or a microbial cell.

In an embodiment, the modification refers to a break in the target sequence, such as a single-strand break/double-strand break in DNA or a single-strand break in RNA.

In some cases, the method further includes making the target nucleic acid in contact with a donor polynucleotide. The donor polynucleotide, a portion of the donor polynucleotide, a copy of the donor polynucleotide, or a portion of the copy of the donor polynucleotide is integrated into the target nucleic acid.

In an embodiment, the modification further includes inserting an edit template (such as an exogenous nucleic acid) into the break.

In an embodiment, the method further includes making an edit template in contact with the target nucleic acid or delivering the edit template to a cell carrying the target nucleic acid. In this embodiment, the method can repair a broken target gene through homologous recombination with an exogenous template polynucleotide. In some embodiments, the repair results in a mutation, including insertion, deletion, or substitution of one or more nucleotides in the target gene. In other embodiments, the mutation results in one or more amino acid changes in a protein expressed by a gene carrying the target sequence.

### Detection (non-specific cleavage)

In another aspect, the present disclosure provides a method for detecting a target nucleic acid in a sample, including: making the sample in contact with the Cas protein, the nucleic acid, the composition, the CIRSPR/Cas system, the vector system, the delivery composition, or the activated CRISPR complex and a single-stranded nucleic acid detector; and detecting a detectable signal generated due to cleavage of the single-stranded nucleic acid detector by the Cas protein to detect the target nucleic acid.

In the present disclosure, the target nucleic acid includes ribonucleotides or deoxyribonucleotides, and the target nucleic acid includes a single-stranded nucleic acid and a double-stranded nucleic acid, such as single-stranded DNA, double-stranded DNA, single-stranded RNA, and double-stranded RNA.

In an embodiment, the target nucleic acid is derived from a sample such as a virus, a bacterium, a microorganism, a soil, a water source, a human body, an animal, or a plant. Preferably, the target nucleic acid is a product of enrichment or amplification by a method such as PCR, NASBA, RPA, SDA, LAMP, HAD, NEAR, MDA, RCA, LCR, or RAM.

In an embodiment, the target nucleic acid is a viral nucleic acid, a bacterial nucleic acid, a disease-related specific nucleic acid such as a specific mutation site or a single nucleotide polymorphism (SNP) site, or a nucleic acid different from a control. Preferably, the virus is a plant virus or an animal virus, such as a papilloma virus, a liver DNA virus, a herpes virus, an adenovirus, a poxvirus, a parvovirus, and a coronavirus. Preferably, the virus is a coronavirus, preferably SARS, SARS-CoV2 (COVID-19), HCoV-229E, HCoV-OC43, HCoV-NL63, HCoV-HKU1, or Mers-Cov.

In the present disclosure, the gRNA has a matching degree of at least 50%, preferably at least 60%, preferably at least 70%, preferably at least 80%, and preferably at least 90% with the target sequence on the target nucleic acid.

In an embodiment, when the target sequence includes one or more characteristic sites (such as specific mutation sites or SNPs), the characteristic sites completely match the gRNA.

In an embodiment, the detection method includes one or more gRNAs with different guide sequences, which target different target sequences.

In the present disclosure, the single-stranded nucleic acid detector includes, but is not limited to, a single-stranded DNA, a single-stranded RNA, a DNA-RNA hybrid, a nucleic acid analogue, a base modifier, and a single-stranded nucleic acid detector with an abasic spacer. The nucleic acid analogue includes, but is not limited to, a locked nucleic acid, a bridged nucleic acid, morpholino, a glycol nucleic acid, a hexitol nucleic acid, a threose nucleic acid, an arabino nucleic acid, 2'-O-methyl RNA, 2'-methoxyacetyl RNA, 2'-fluoro RNA, 2'-amino RNA, 4'-thio RNA, and a combination thereof, including optional ribonucleotide or deoxyribonucleotide residues.

In the present disclosure, the detectable signal is detected in the following ways: vision-based detection, sensor-based detection, color detection, fluorescence signal-based detection, gold nanoparticle-based detection, fluorescence polarization, colloidal phase change/dispersion, electrochemical detection, and semiconductor-based detection.

In the present disclosure, preferably, two termini of the single-stranded nucleic acid detector are provided with a fluorophore and a quencher respectively. When the single-stranded nucleic acid detector is cleaved, a detectable fluorescence signal can be presented. The fluorophore is selected from any one or more of FAM, FITC, VIC, JOE, TET, CY3, CY5, ROX, Texas Red, or LC RED460. The quencher is selected from any one or more of BHQ1, BHQ2, BHQ3, Dabcyl, or Tamra.

In other embodiments, a 5' terminus and a 3' terminus of the single-stranded nucleic acid detector are provided with different labeling molecules. The single-stranded nucleic acid detector is subjected to a colloidal gold test before and after being cleaved by the Cas protein. The single-stranded nucleic acid detector shows different chromogenic results on colloidal gold detection line and control line before and after being cleaved by the Cas protein.

In some embodiments, the method for detecting a target nucleic acid may further include: comparing a level of the detectable signal with a reference signal level, and determining an amount of the target nucleic acid in the sample based on the level of the detectable signal.

In some embodiments, the method for detecting a target nucleic acid may further include: using an RNA reporter nucleic acid and a DNA reporter nucleic acid (such as a fluorescence color) on different channels, measuring signal levels of the RNA and DNA reporter molecules and amounts of the target nucleic acid in the RNA and DNA reporters to determine a level of the detectable signal, and sampling based on a combined (such as minimum or product) level of the detectable signal.

In an embodiment, the target gene is present in a cell.

In an embodiment, the cell is a prokaryotic cell.

In an embodiment, the cell is a eukaryotic cell.

In an embodiment, the cell is an animal cell.

In an embodiment, the cell is a human cell.

In an embodiment, the cell is a plant cell, such as a cell possessed by a cultivated plant (such as cassava, corn, sorghum, wheat, or rice), an alga, a tree, or a vegetable.

In an embodiment, the target gene is present in a nucleic acid *in vitro* (such as a plasmid).

In an embodiment, the target gene is present in a plasmid.

### Terms and definitions

In the present disclosure, unless otherwise specified, the scientific and technical terms used herein have the meanings commonly understood by those skilled in the art. In addition, the operation procedures used herein such as molecular genetics, nucleic acid chemistry, chemistry, molecular biology, biochemistry, cell culture, microbiology, cell biology, genomics, and DNA recombination are routine procedures widely used in the corresponding fields. Moreover, in order to well explain the present disclosure, the definitions and explanations of related terms are provided below.

The nucleic acid cleavage or the cleavage of a nucleic acid herein includes: a DNA or RNA break in a target nucleic acid caused by the Cas enzyme described herein (Cis cleavage), and a DNA or RNA break in a side-branch nucleic acid substrate (single-stranded nucleic acid substrate) (namely, non-specific or non-targeting Trans cleavage). In some embodiments, the cleavage refers to a double-stranded DNA break. In some embodiments, the cleavage refers to a single-stranded DNA break or a single-stranded RNA break.

### CRISPR system

The terms "CRISPR-Cas system" or "CRISPR system" used herein can be used interchangeably and have the meaning commonly understood by those skilled in the art. The CRISPR-Cas system or the CRISPR system usually includes a transcription product or other elements related to the expression of a Cas gene, or a transcription product or other elements capable of guiding an activity of the Cas gene.

### CRISPR/Cas complex

As used herein, the term "CRISPR/Cas complex" refers to a complex produced through the binding of a gRNA or mature crRNA to the Cas protein. The CRISPR/Cas complex includes a direct repeat that hybridizes with a guide sequence of the target sequence and binds to the Cas protein. The CRISPR/Cas complex can recognize and cleave a polynucleotide capable of hybridizing with the gRNA or mature crRNA.

### gRNA

As used herein, the terms "gRNA", "mature crRNA", and "guide sequence" can be used interchangeably and have the meanings commonly understood by those skilled in the art. Generally, gRNA can include a direct repeat and a guide sequence, or is essentially composed of or is composed of a direct repeat and a guide sequence.

In some cases, the guide sequence is any polynucleotide sequence that shows sufficient complementarity with a target sequence to hybridize with the target sequence and guide the specific binding of the CRISPR/Cas complex to the target sequence. In an embodiment, under the optimal alignment, a complementarity degree between the guide sequence and a corresponding target sequence is at least 50%, at least 60%, at least 70%, at least 80%, at least 90%, at least 95%, or at least 99%. The determination of the optimal alignment is within the competence of those of ordinary skill in the art. For example, there are published and commercially-available alignment algorithms and programs, including but not limited to Smith-Waterman, Bowtie, Geneious, Biopython, and SeqMan in ClustalW and matlab.

### Target sequence

A target sequence refers to a polynucleotide targeted by a guide sequence in gRNA, such as a sequence complementary to the guide sequence. The hybridization between the target sequence and the guide sequence will promote the production of a CRISPR/Cas complex (including a Cas protein and a gRNA). The complete complementarity is not necessary, as long as there is sufficient complementarity to cause hybridization and promote the production of a CRISPR/Cas complex.

The target sequence can include any polynucleotide, such as DNA or RNA. In some cases, the target sequence is located inside or outside a cell. In some cases, the target sequence is located in the nucleus or cytoplasm of a cell. In some cases, the target sequence may be located in an organelle of a eukaryotic cell, such as a mitochondrion or a chloroplast. A sequence or a template that can be recombined into a target gene locus with the target sequence is called "edit template" or "edit polynucleotide" or "edit sequence". In an embodiment, the edit template is an exogenous nucleic acid. In an embodiment, the recombination refers to homologous recombination.

In the present disclosure, the "target sequence" or "target polynucleotide" or "target nucleic acid" can be any endogenous or exogenous polynucleotide for a cell (such as a eukaryotic cell). For example, the target polynucleotide may be a polynucleotide present in the nucleus of a eukaryotic cell. The target polynucleotide may be a sequence encoding a gene product (such as a protein) or a non-coding sequence (such as a regulatory polynucleotide or useless DNA). In some cases, the target sequence should be related to PAM.

### Single-stranded nucleic acid detector

The single-stranded nucleic acid detector of the present disclosure refers to a sequence with 2 to 200 nucleotides, preferably 2 to 150 nucleotides, preferably 3 to 100 nucleotides, preferably 3 to 30 nucleotides, preferably 4 to 20 nucleotides, and more preferably 5 to 15 nucleotides. Preferably, the single-stranded nucleic acid detector is a single-stranded DNA molecule, a single-stranded RNA molecule, or a single-stranded DNA-RNA hybrid.

Two termini of the single-stranded nucleic acid detector include different reporter groups or labeling molecules. When the single-stranded nucleic acid detector is in an initial state (that is, when the single-stranded nucleic acid detector is not cleaved), no reporter signal is presented. When the single-stranded nucleic acid detector is cleaved, a detectable signal is presented. That is, a detectable difference is presented before and after cleavage.

In an embodiment, the reporter groups or labeling molecules include fluorophores and quenchers. The fluorophores are selected from any one or more of FAM, FITC, VIC, JOE, TET, CY3, CY5, ROX, Texas Red, or LC RED460. The quenchers are selected from any one or more of BHQ1, BHQ2, BHQ3, Dabcyl, or Tamra.

In an embodiment, the single-stranded nucleic acid detector has a first molecule (such as FAM or FITC) linked to a 5' terminus and a second molecule (such as biotin) linked to a 3' terminus. A reaction system with the single-stranded nucleic acid detector is used in combination with a flow strip to detect a target nucleic acid (preferably, colloidal gold detection). The flow strip is designed to have two capture lines. An antibody to bind to the first molecule (namely, an anti-first molecule antibody) is arranged at a sample contact end (colloidal gold), an antibody to bind to the anti-first molecule antibody is arranged at a first line (control line), and an antibody to bind to a second molecule (namely, an anti-second molecule antibody, such as avidin) is arranged at a second line (test line). As a reaction proceeds along the strip, the anti-first molecule antibody binds to the first molecule and carries a cleaved or uncleaved oligonucleotide to the capture line. A cleaved reporter will bind to the antibody binding to the anti-first molecule antibody at the first capture line, and an uncleaved reporter will bind to the anti-second molecule antibody at the second capture line. The binding of the reporter group to each line will result in a strong readout/signal (such as a color). As the increased reporters are cleaved, the increased signals will accumulate at the first capture line, and the decreased signals will appear at the second line. In some aspects, the present disclosure relates to a use of the flow strip as described herein in detection of a nucleic acid. In some aspects, the present disclosure relates to a method for detecting a nucleic acid using a flow strip as defined herein, such as a (lateral) flow test or a (lateral) flow immunochromatographic assay. In some aspects, the molecules in the single-stranded nucleic acid detector can be used instead of each other, or positions of the molecules can be changed. As long as there is the same or similar reporting principle as or to the present disclosure, an improved method is also included in the present disclosure.

The detection method of the present disclosure can be used for quantitative detection of a target nucleic acid to be detected. The quantitative detection index can be quantified according to a signal intensity of a reporter group, for example, according to a luminous intensity of a fluorophore or according to a width of a chromogenic band.

### WT

As used herein, the term "WT" has the meaning commonly understood by those skilled in the art, and indicates the typical form of an organism, a strain, or a gene, or a characteristic to distinguish a natural form of the organism, strain, or gene from a mutant or variant form thereof, which can be isolated from a natural source and is not artificially modified intentionally.

### Derivatization

As used herein, the term "derivatization" refers to the chemical modification to an amino acid, a polypeptide, or a protein, where one or more substituents have been covalently linked to the amino acid, the polypeptide, or the protein. The substituents can also be referred to as side chains.

A derivatized protein is a derivative of a protein. Generally, the derivatization of a protein will not adversely affect the desired activity of the protein (for example, an activity to bind to gRNA, an endonuclease activity, and an activity to bind to a specific site of a target sequence and cleave the target sequence under the guidance of gRNA). That is, a derivative of a protein has the same activity as the protein.

### Derivatized protein

A derivatized protein, also known as "protein derivative", refers to a modified form of a protein. For example, one or more amino acids of the protein can be deleted, inserted, modified, and/or substituted.

### Non-natural

As used herein, the terms "non-natural" and "engineered" can be used interchangeably and refer to human intervention. When these terms are used to describe a nucleic acid or a polypeptide, it means that the nucleic acid or polypeptide is at least substantially isolated from a natural source or separated from at least another component binding to the nucleic acid or polypeptide in nature.

### Orthologue, ortholog

As used herein, the term "orthologue" or "ortholog" has the meaning commonly understood by those skilled in the art. As a further guide, an "orthologue" of the protein described herein refers to a protein of a different species, which implements the same function as or the similar function to the protein.

### Identity

As used herein, the term "identity" refers to the sequence matching between two polypeptides or between two nucleic acids. When specified positions in two sequences to be compared are occupied by a same base or amino acid monomer subunit (for example, a specified position in each of two DNA molecules is occupied by adenine, or a specified position in each of two peptides is occupied by lysine), the molecules are identical at the position. The "percent identity" between two sequences is a function of a number of matched positions shared by the two sequences/a number of compared positions × 100. For example, if 6 of 10 positions in a sequence match corresponding positions in another sequence, then the two sequences have an identity of 60%. For example, DNA sequences CTGACT and CAGGTT have an identity of 50% (3 of 6 positions are matching). Generally, the comparison is conducted when two sequences are aligned to produce the maximum identity. Such alignment can be allowed by using, for example, a method of Needleman et al. (1970) J. Mol. Biol. 48: 443-453 that can be conveniently implemented by a computer program such as Align program (DNAstar, Inc.). The percent identity between two amino acid sequences can also be determined by using an algorithm of E. Meyers and W. Miller (Comput. Appl Biosci., 4:11-17 (1988) integrated into the ALIGN program (version 2.0), a PAM120 weight residue table, a gap length penalty of 12, and a gap penalty of 4. In addition, the percent identity between two amino acid sequences can be determined by using Needleman and Wunsch (J MoI Biol. 48:444-453 (1970)) algorithms in the GAP program integrated into the GCG software package (available on www.gcg.com), a Blossum 62 matrix or a PAM250 matrix, a gap weight of 16, 14, 12, 10, 8, 6, or 4, and a length weight of 1, 2, 3, 4, 5, or 6.

### Vector

The term "vector" refers to a nucleic acid that can deliver another nucleic acid linked thereto. The vector includes, but is not limited to, a single-stranded, double-stranded, or partially double-stranded nucleic acid; a nucleic acid with one or more free ends or without free ends (such as circular); DNA, RNA, or a nucleic acid of both; and other diverse polynucleotides known in the art. The vector can be introduced into a host cell through transformation, transduction, or transfection, such that a genetic material element carried by the vector can be expressed in the host cell. A vector can be introduced into a host cell to produce a transcript, a protein, or a peptide, including the protein, the fusion protein, the isolated nucleic acid, etc. (for example, a CRISPR transcript, such as a nucleic acid transcript, a protein, or an enzyme) described herein. A vector may include a variety of elements to control the expression, including but not limited to a promoter sequence, a transcription initiation sequence, an enhancer sequence, a selection element, and a reporter gene. In addition, the vector may also include a replication origin.

A type of a vector is a plasmid, which refers to a circular double-stranded DNA loop where an additional DNA fragment can be inserted, for example, by a standard molecular cloning technique.

Another type of a vector is a viral vector. A virus-derived DNA or RNA sequence is present in a vector for packaging a virus (such as a retrovirus, a replication-defective retrovirus, an adenovirus, a replication-defective adenovirus, and an adeno-associated virus). The viral vector also includes a polynucleotide carried by a virus to be transfected into a host cell. Some vectors (for example, bacterial vectors with bacterial replication origins and episomal mammalian vectors) can autonomously replicate in a host cell into which the vectors are introduced.

Other vectors (such as non-episomal mammalian vectors) will be integrated into a genome of a host cell after being introduced into the host cell, and thus will replicate with the genome of the host cell. Moreover, some vectors can guide the expression of genes operably linked thereto. Such vectors are referred to as "expression vectors" herein.

### Host cell

As used herein, the term "host cell" refers to a cell that can be introduced with a vector, including, but not limited to, a prokaryotic cell such as *Escherichia coli* or *Bacillus subtilis,* and a eukaryotic cell such as a microbial cell, a fungal cell, an animal cell, and a plant cell.

Those skilled in the art will understand that the design of an expression vector may depend on factors such as the selection of a host cell to be transformed and a desired expression level.

### Regulatory element

As used herein, the term "regulatory element" is intended to include a promoter, an enhancer, an IRES, and other expression control elements (for example, a transcriptional termination signal, such as a polyadenylation signal and a poly-U sequence). The detailed description can be seen in Goeddel, "GENE EXPRESSION TECHNOLOGY: METHODS IN ENZYMOLOGY" 185, Academic Press, San Diego, California (1990). In some cases, the regulatory element includes sequences that guide the constitutive expression of a nucleotide sequence in many types of host cells and sequences that guide the expression of the nucleotide sequence only in some host cells (such as a tissue-specific regulatory sequence). A tissue-specific promoter can mainly guide the expression in a desired tissue of interest, such as muscles, neurons, bones, skin, blood, specific organs (such as liver and pancreas), or specific cell types (such as lymphocytes). In some cases, a regulatory element can also guide the expression in a time-dependent manner (such as in a cell cycle-dependent or developmental stage-dependent manner), which may be or may not be tissue or cell type-specific. In some cases, the term "regulatory element" covers enhancer elements, such as WPRE; a CMV enhancer; an R-U5' fragment in LTR of HTLV-I ((Mol.Cell.Biol., Vol 8 (1): 466-472, 1988); an SV40 enhancer; and an intron sequence between exons 2 and 3 of rabbit β-globin (Proc. Natl. Acad. Sci. USA., Vol. 78 (3): 1527-31, 1981).

### Promoter

As used herein, the term "promoter" has the meaning well known to those skilled in the art, and refers to a non-coding nucleotide sequence that is located upstream of a gene and can promote the expression of a downstream gene. A constitutive promoter is a nucleotide sequence that will result in the generation of a gene product in a cell under most or all physiological conditions of the cell after the promoter is operably linked to a polynucleotide encoding or defining the gene product. An inducible promoter is a nucleotide sequence that will cause the generation of a gene product in a cell only when there is an inducer corresponding to the promoter in the cell after the promoter is operably linked to a polynucleotide encoding or defining the gene product. A tissue-specific promoter is a nucleotide sequence that will cause the generation of a gene product in a cell basically only when the cell is a cell of a tissue type corresponding to the promoter after the promoter is operably linked to a polynucleotide encoding or defining the gene product.

### NLS

"NLS" is an amino acid sequence that tags a protein for import into a nucleus through nuclear transport, that is, a protein with NLS is transported to the nucleus. Typically, NLS includes positively-charged Lys or Arg residues that are exposed on a surface of a protein. Exemplary NLS includes, but is not limited to, SV40 large T antigen, EGL-13, c-Myc, and TUS protein. In some embodiments, the NLS includes a PKKKRKV sequence. In some embodiments, the NLS includes an AVKRPAATKKAGQAKKKKLD sequence. In some embodiments, the NLS includes a PAAKRVKLD sequence. In some embodiments, the NLS includes an MSRRRKANPTKLSENAKKLAKEVEN sequence. In some embodiments, the NLS includes a KLKIKRPVK sequence. Other NLS includes, but is not limited to, an acidic M9 domain of hnRNP A1, and sequences KIPIK and PY-NLS in a yeast transcription repressor Matα2.

### Operably linked

As used herein, the term "operably linked" means that a nucleotide sequence of interest is linked to one or more regulatory elements in a manner that allows the expression of the nucleotide sequence (for example, in an *in vitro* transcription/translation system or in a host cell when the vector is introduced into the host cell).

### Complementarity

As used herein, the term "complementarity" refers to the ability of a nucleic acid to form one or more hydrogen bonds with another nucleic acid by means of the traditional Watson-Crick or another non-traditional form. The complementarity percentage refers to a percentage of residues in a first nucleic acid that can form hydrogen bonds (such as Watson-Crick base pairing) with a second nucleic acid (such as 5, 6, 7, 8, 9, and 10 of 10, namely a complementarity of 50%, 60%, 70%, 80%, 90%, and 100%). The "completely complementary" means that all continuous residues of a nucleic acid sequence form hydrogen bonds with a same number of continuous residues in another nucleic acid sequence. As used herein, the "substantially complementary" means that there is a complementarity degree of at least 60%, 65%, 70%, 75%, 80%, 85%, 90%, 95%, 97%, 98%, 99%, or 100% in a region with 8, 9, 10, 11, 12, 13, 14, 15, 16, 17, 18, 19, 20, 21, 22, 23, 24, 25, 30, 35, 40, 45, 50, or more nucleotides, or means that two nucleic acids can hybridize under stringent conditions.

### Stringent conditions

As used herein, the "stringent conditions" for hybridization refer to conditions under which a nucleic acid showing complementarity with a target sequence mainly hybridizes with the target sequence and substantially does not hybridize with a non-target sequence. Stringent conditions are usually sequence-dependent and vary depending on many factors. Generally, the longer the sequence, the higher the temperature at which the sequence specifically hybridizes with a corresponding target sequence.

### Hybridization

The term "hybridization" or "complementary" or "substantially complementary" means that a nucleic acid (such as RNA and DNA) includes a nucleotide sequence enabling the noncovalent binding, that is, the nucleic acid can form base pairs and/or G/U base pairs with another nucleic acid in a sequence-specific, anti-parallel manner (that is, the nucleic acid specifically binds to a complementary nucleic acid), "annealing" or "hybridizing".

The hybridization requires that two nucleic acids include complementary sequences. There may be mismatching between bases. Suitable conditions for hybridization between two nucleic acids depend on the length and complementarity degree of the nucleic acids, which are variables well known in the art. Typically, a hybridizable nucleic acid includes 8 or more nucleotides (such as 10 or more nucleotides, 12 or more nucleotides, 15 or more nucleotides, 20 or more nucleotides, 22 or more nucleotides, 25 or more nucleotides, or 30 or more nucleotides).

It should be understood that a sequence of a polynucleotide does not need to be 100% complementary to a sequence of a corresponding target nucleic acid for specific hybridization. A polynucleotide has a complementarity of 60% or more, 65% or more, 70% or more, 75% or more, 80% or more, 85% or more, 90% or more, 95% or more, 98% or more, 99% or more, 99.5% or more, or 100% with a sequence of a target region in a target nucleic acid sequence with which the polynucleotide is to hybridize.

The hybridization of a target sequence with gRNA means that the target sequence and a nucleic acid sequence of the gRNA have a complementarity of at least 60%, 70%, 75%, 80%, 85%, 90%, 91%, 92%, 93%, 94%, 95%, 96%, 97%, 98%, 99%, or 100%, and thus can be hybridized to produce a complex, or means that at least 12, 15, 16, 17, 18, 19, 20, 21, 22, or more bases in the target sequence are complementary to and paired with the respective bases in the nucleic acid sequence of the gRNA, and thus the two sequences can be hybridized to produce a complex.

### Expression

As used herein, the term "expression" refers to a process by which a DNA template is transcribed into a polynucleotide (such as mRNA or another RNA transcript) and/or a process by which the transcribed mRNA is subsequently translated into a peptide, a polypeptide, or a protein. The transcript and the encoded polypeptide can be collectively referred to as "gene product". If a polynucleotide is derived from genomic DNA (gDNA), the expression can include the splicing of mRNA in a eukaryotic cell.

### Linker

As used herein, the term "linker" refers to a linear polypeptide produced by linking a plurality of amino acid residues through peptide bonds. The linker of the present disclosure may be an artificially-synthesized amino acid sequence, or a natural polypeptide sequence, such as a polypeptide with a hinge domain function. Such linker polypeptides are well known in the art (see, for example, Holliger, P. et al. (1993) Proc. Natl. Acad. Sci. USA 90: 6444-6448; and Poljak, R. J. et al. (1994) Structure 2: 1121-1123).

### Treatment

As used herein, the term "treatment" refers to treating or curing a disease, delaying the onset of symptoms of a disease, and/or delaying the development of a disease.

### Subject

As used herein, the term "subject" includes, but is not limited to, various animals, plants, and microorganisms.

### Animal

For example, the animal may be a mammal, such as bovine, equine, sheep, swine, canine, feline, leporid, rodent (such as a mouse or rat), non-human primate (such as a macaque or cynomolgus monkey), or human. In some embodiments, the subject (such as human) suffers from a disorder (such as a disorder caused by a disease-related gene defect).

### Plant

The term "plant" should be understood as any differentiated multicellular organism capable of conducting photosynthesis, including: any crop plants at a mature or developmental stage, especially monocotyledonous or dicotyledonous plants; vegetable crops including artichoke, turnip cabbage, arugula, leek, asparagus, lettuce (such as head lettuce, leaf lettuce, and romaine lettuce), bok choy, malanga, melons (such as cantaloupe, watermelon, crenshaw melon, honeydew melon, and Roman cantaloupe), rape crops (such as Brussels sprout, cabbage, cauliflower, broccoli, borecole, kale, Chinese cabbage, and bok choy), cardoon, carrot, napa, okra, onion, celery, parsley, chickpea, parsnip, chicory, pepper, *Solanum tuberosum,* gourd (such as marrow squash, cucumber, zucchini, cushaw, and pumpkin), radish, dried ball onion, rutabaga, purple eggplant (also known as eggplant), salsify, lettuce, shallot, endive, garlic, spinach, green onion, cushaw, greens, beets (sugar beets and fodder beets), sweet potato, Swiss chard, wasabi, tomato, turnip, and spices; fruits and/or vine crops such as apple, apricot, cherry, nectarine, peach, pear, plum, prune, cherry, quince, almond, chestnut, hazelnut, pecan, pistachio, walnut, citrus, blueberry, boysenberry, cranberry, currant, loganberry, raspberry, strawberry, blackberry, grape, avocado, banana, kiwi, persimmon, pomegranate, pineapple, tropical fruit, pome, melon, mango, papaya, and lychee; field crops, such as clover, alfalfa, evening primrose, meadowfoam, corn/maize (forage corn, sweet corn, and popcorn), lupulus, jojoba, peanut, rice, safflower, small grain crops (*Hordeum vulgare,* oat, rye, *Triticum aestivum,* etc.), *Sorghum bicolor, Nicotiana tabacum,* kapok, legumes (beans, lentil, pea, and *Glycine max*)*,* oil plants (canola, leaf mustard, olive, sunflower, coconut, castor oil plant, cocoa bean, and groundnut), *Arabidopsis,* fiber plants (cotton, flax, hemp, and jute), *Lauraceae* (cinnamon or camphor), or a plant such as coffee, sugar cane, tea, and natural rubber plants; and/or bedding plants such as a flowering plant, cactus, a succulent plant, and/or an ornamental plant, and trees such as forests (broad-leaved and evergreen trees, such as conifers), fruit trees, ornamental trees, nut-bearing trees, shrubs, and other seedlings.

### Advantageous effects of the present disclosure

The present disclosure improves an activity of a Cas12j 19 protein through a mutation, which has a promising application prospect.

Embodiments of the present disclosure will be described in detail below with reference to accompanying drawings and examples, but those skilled in the art will understand that the following accompanying drawings and examples are used to merely illustrate the present disclosure rather than limit the scope of the present disclosure. Through the following detailed description of accompanying drawings and preferred embodiments, various objects and advantageous aspects of the present disclosure will become apparent to those skilled in the art.

### DESCRIPTION OF THE DRAWINGS

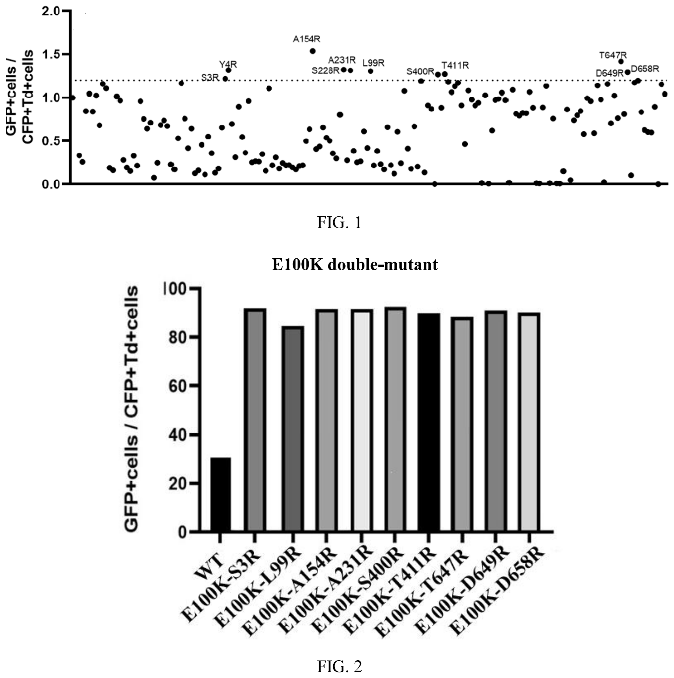

FIG. 1 shows validation results of editing efficiencies of single-mutant Cas proteins, where y-coordinates represent proportions of green fluorescent protein (GFP)-positive cells in cyan fluorescent protein (CFP)- and red fluorescent protein (RFP)-positive populations detected by flow cytometry, namely, editing efficiencies, and x-coordinates represent different mutants;
FIG. 2 shows editing activities of Cas proteins including E100K, where y-coordinates represent proportions of GFP-positive cells in CFP- and RFP-positive populations detected by flow cytometry, namely, editing efficiencies, and x-coordinates represent different mutants;

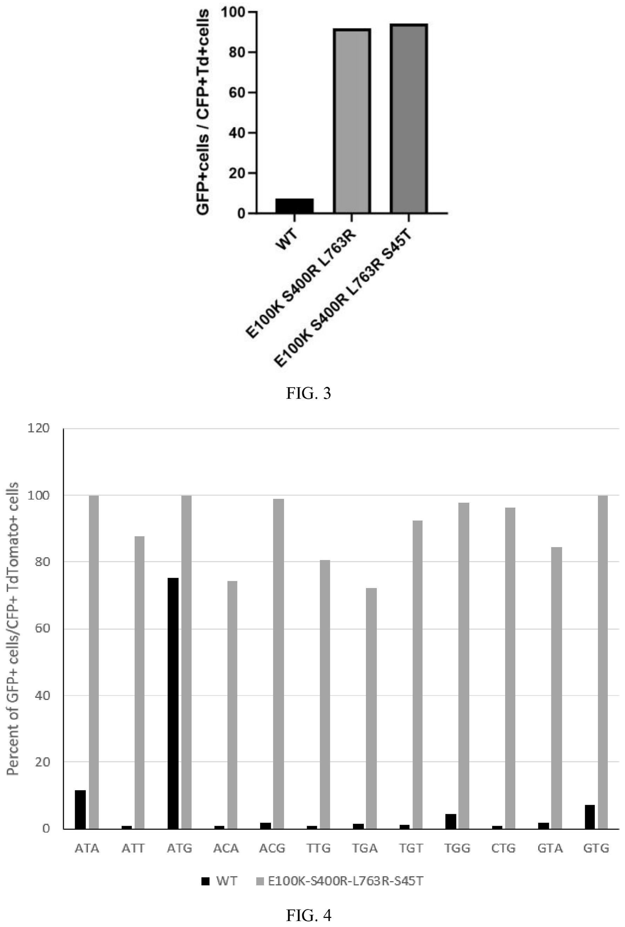

FIG. 3 shows validation results of editing activities of multi-mutant Cas proteins, where y-coordinates represent proportions of GFP-positive cells in CFP- and RFP-positive populations detected by flow cytometry, namely, editing efficiencies, and x-coordinates represent different mutants; and
FIG. 4 shows recognition results of multi-mutant Cas proteins for different PAMs, where y-coordinates represent proportions of GFP-positive cells in CFP- and RFP-positive populations detected by flow cytometry, namely, editing efficiencies; x-coordinates represent different mutants; WT represents WT Cas12j 19; and E100K-S400R-L763R-S45T represents a quadruple-mutant protein.

### SPECIFIC IMPLEMENTATIONS

The following examples are only used to describe rather than limit the present disclosure. Unless otherwise specified, the experiments and methods described in the examples are basically conducted in accordance with the conventional methods well known in the art and described in various references. For example, the conventional techniques such as immunology, biochemistry, chemistry, molecular biology, microbiology, cell biology, genomics, and DNA recombination used in the present disclosure can be found in *"*MOLECULAR CLONING: A LABORATORY MANUAL", Sambrook, Fritsch, and Maniatis, edition 2 (1989); *"*CURRENT PROTOCOLS IN MOLECULAR BIOLOGY" (edited by F. M. Ausubel et al., (1987)); and *"*METHODS IN ENZYMOLOGY" series (Academic Press Corporation): *"*PCR 2: A PRACTICAL APPROACH (edited by M. J. MacPherson, B. D. Hames, and G. R. Taylor (1995)), *A*NTIBODIES, A LABORATORY MANUAL edited by Harlow and Lane (1988), and *"*ANIMAL CELL CULTURE" (edited by R. I. Freshney (1987)).

In addition, if no specific conditions are specified in the examples, the examples will be conducted according to conventional conditions or the conditions recommended by the manufacturer. All of the used reagents or instruments which are not specified with manufacturers are conventional commercially-available products. Those skilled in the art know that the present disclosure is described by way of examples in the embodiments, and the examples are not intended to limit the protection scope of the present disclosure. All publications and other references mentioned herein are incorporated herein by reference in their entireties.

### Example 1 Acquisition of Cas mutant proteins

The applicants predicted possible key amino acid sites in the known Cas protein (which was referred to as Cas12j.19 in CN111770992B and was referred to as Cas12j19 in this example) to affect a biological function of the Cas protein through bioinformatics, and conducted a mutation at the amino acid sites to produce Cas mutant proteins with improved editing activities. Specifically, a coding sequence for Cas12j19 was codon-optimized and synthesized. For WT Cas12j19, an amino acid sequence is shown in SEQ ID No. 1 and a nucleic acid sequence is shown in SEQ ID No. 2. Potential amino acids in Cas12j19 to bind to a target sequence were subjected to site-directed mutagenesis by a bioinformatics method.
SEQ ID No. 1: 
MPSYKSSRVLVRDVPEELVDHYERSHRVAAFFMRLLLAMRREPYSLRMRDGTEREV
DLDETDDFLRSAGCEEPDAVSDDLRSFALAVLHQDNPKKRAFLESENCVSILCLEKSASG
TRYYKRPGYQLLKKAIEEEWGWDKFEASLLDERTGEVAEKFAALSMEDWRRFFAARDP
DDLGRELLKTDTREGMAAALRLRERGVFPVSVPEHLDLDSLKAAMASAAERLKSWLA
CNQRAVDEKSELRKRFEEALDGVDPEKYALFEKFAAELQQADYNVTKKLVLAVSAKFPA
TEPSEFKRGVEILKEDGYKPLWEDFRELGFVYLAERKWERRRGGAAVTLCDADDSPIKV
RFGLTGRGRKFVLSAAGSRFLITVKLPCGDVGLTAVPSRYFWNPSVGRTTSNSFRIEFTKR
TTENRRYVGEVKEIGLVRQRGRYYFFIDYNFDPEEVSDETKVGRAFFRAPLNESRPKPKD
KLTVMGIDLGINPAFAFAVCTLGECQDGIRSPVAKMEDVSFDSTGLRGGIGSQKLHREMH
NLSDRCFYGARYIRLSKKLRDRGALNDIEARLLEEKYIPGFRIVHIEDADERRRTVGRTV
KEIKQEYKRIRHQFYLRYHTSKRDRTELISAEYFRMLFLVKNLRNLLKSWNRYHWTTGD
 
RERRGGNPDELKSYVRYYNNLRMDTLKKLTCAIVRTAKEHGATLVAMENIQRVDRDDE
VKRRKENSLLSLWAPGMVLERVEQELKNEGILAWEVDPRHTSQTSCITDEFGYRSLVAK
DTFYFEQDRKIHRIDADVNAAINIARRFLTRYRSLTQLWASLLDDGRYLVNVTRQHERAY
LELQTGAPAATLNPTAEASYELVGLSPEEEELAQTRIKRKKREPFYRHEGVWLTREKHRE
QVHELRNQVLALGNAKIPEIRT
SEQ ID No. 2:

atgcccagctacaagagcagcagagtgctggtgagGgacgtgccagaggagctggtggaccactacgaaagaagccacagggt
ggccgccttcttcatgagactgctgctggccatgagaagagagccctacagcctgagaatgagGgacggaaccgagagagaggtggac
ctggacgaaaccgacgacttcctgagaagcgccggatgcgaggagccagacgccgtgtctgacgacctgagaagcttcgccctggccgt
gctgcaccaggacaacccaaagaagagagcctttctggagagcgagaactgcgtgtcaatcctgtgtctggagaaaagcgcctccggca
ccagatactacaaaagacccggctaccagctgctgaagaaggccatcgaggaggagtggggatgggacaagttcgaggcctctctgctg
gacgagagaacaggagaagtggccgagaagttcgccgccctgagcatggaagactggagaagattcttcgccgccagagaccccgac
gatctgggaagagagctgctgaagaccgacaccagagaaggcatggccgccgccctgagactgagagaaagaggagtgttccccgtga
gcgtgcccgagcacctggacctggacagcctgaaggccgccatggccagcgccgctgaaagactgaagtcatggctggcctgtaacca
gcgcgccgtggacgagaagagcgaactgagaaagagattcgaggaggccctggacggagtggaccctgaaaagtatgccctgtttgag
aagttcgctgccgagctgcagcaggccgactacaacgtgaccaagaaactggtgctggccgtgagcgccaagttccccgctaccgagcc
tagcgagtttaagagaggagtggagatcctgaaggaagatggctacaagcccctgtgggaggactttagagagctgggctttgtgtatctg
gccgagagaaagtgggaaagaagaagaggcggagccgccgtgaccctgtgtgacgctgacgacagccctatcaaggtgagatttggac
tgactggcagaggcagaaagtttgtgctgtccgccgccgggagcagattcctgatcacagtgaagctgccctgcggcgatgtgggactga
ccgccgtgcccagcaggtacttttggaacccctcagtgggcagaaccacctccaactcttttagaattgagttcaccaagcggaccaccga
gaatcgcaggtacgtgggcgaggtgaaagagattggcctggtgaggcagagaggcagatactattttttcatcgactacaacttcgaccca
gaggaggtgagcgacgagaccaaggtgggcagagccttcttcagagcccccctgaacgagtcaagacctaagccaaaggacaagctga
ccgtgatggggattgacctgggcattaacccagcctttgcctttgccgtgtgcaccctgggcgaatgccaggacggaatccggagccccgt
ggccaaaatggaggacgtgagcttcgactctaccggactgagaggcggaatcggcagccagaagctgcacagagagatgcacaacctg
agcgacagatgcttttacggcgccagatacattagactgagcaagaagctgagggacagaggagctctgaacgatatcgaggctagactg
ctggaagagaagtacatccccggctttagaattgtgcacattgaggacgccgacgagagaagaagaaccgtgggaagaacagtgaaaga
aatcaaacaggagtacaaaagaatcagacaccagttctacctgaggtaccacaccagcaagagggacagaacagagctgattagcgccg
agtacttcagaatgctgttcctggtgaagaacctgagaaacctgctgaagagctggaacagataccactggacaaccggcgacagagaaa
gaagaggagggaacccagacgagctgaagagctatgtgagatactataacaatctgagaatggacaccctgaagaaactgacctgcgcc
atcgtgaggaccgccaaggaacacggagccaccctggtggccatggagaacatccagagagtggacagGgacgacgaggtgaaaag
aagaaaggaaaatagcctgctgagcctgtgggcccccggaatggtgctggagagagtggagcaggagctgaagaacgagggaatcct
ggcctgggaggtggacccaagacatacaagtcagacaagctgcatcaccgacgaatttggctacagaagcctggtggccaaggacacct
tctactttgaacaggacagaaaaatccacagaatcgacgccgacgtgaacgccgccatcaacatcgcccgccgcttcctgacccggtaca
ggagcctgacccagctgtgggccagcctgctggacgacggcagatacctggtgaacgtgaccagacagcacgagagagcctacctgga
actgcagaccggcgcccccgccgctacactgaaccctaccgccgaagccagctacgaactggtgggactgagccccgaagaagaaga

gctggcccagaccagaatcaaacgcaaaaagagagaaccattctacagacacgaaggcgtgtggctgacaagagagaagcacagaga
gcaggtgcacgaactgagaaaccaggtgctggccctgggcaacgccaagatccccgaaatcagaacc

Variants of the Cas protein were produced through site-directed mutagenesis based on PCR. A specific method was as follows: A DNA sequence for the Cas12j19 protein was designed as two parts with a mutation site as a center, and two pairs of primers were designed to amplify the two parts of the DNA sequence, respectively. A sequence to be mutated was introduced into the primers. Finally, two amplified fragments were loaded on a pcDNA3.3-eGFP vector through Gibson cloning. A combination of mutants was constructed by splitting DNA for the Cas12j19 protein into a plurality of segments and then conducting PCR and Gibson clone. TransStart FastPfu DNA Polymerase (including 2.5 mM dNTPs) was adopted as a fragment amplification kit. A specific experimental procedure was detailed in the manual. FastPure^{®} Gel DNA Extraction Mini Kit was adopted as a gel recovery kit. A specific experimental procedure was detailed in the manual. pEASY-Basic Seamless Cloning and Assembly Kit (CU201-03) was adopted as a kit for vector construction. A specific experimental procedure was detailed in the manual.

Based on the above amino acid mutation sites, the WT Cas12j19 protein and single-mutant proteins with mutations at different amino acid sites were produced.

### Example 2 Verification of editing activities of Cas mutant proteins

A fluorescent reporter system for verification of Cas 12j 19 cleavage was constructed with reference to the literature (Yang, Yi, et al. "Highly efficient and rapid detection of the cleavage activity of Cas9/gRNA via a fluorescent reporter." Applied biochemistry and biotechnology 180.4 (2016): 655-667.). In this fluorescent reporter system, a fluorescent reporter vector carried a red fluorescent protein (RFP) and non-luminous GFFP, and a Cas vector carried CFP. A target site FUT8-3: ATGGAGGCTGTCTACAATGGGGA on a GFFP sequence was selected for testing, where an underlined part was a PAM sequence. A DR sequence was GUGCUGCUGUCUCCCAGACGGGAGGCAGAACUGCAC. 48 h after CHO cells were transfected, a proportion of GFP-positive cells in a CFP- and RFP-positive cell population was determined by FACS to determine an editing efficiency. Different mutants obtained in Example 1 were selected for testing.

FIG. 1 shows editing efficiencies of different Cas proteins, where an editing efficiency of the WT Cas12j19 protein (WT, as shown in SEQ ID No. 1) is set as 1, and different points represent single-mutant Cas proteins with mutations at different single amino acid sites. In FIG. 1, a dashed line represents an editing efficiency 1.3 times the editing efficiency of the WT Cas12j19 protein. According to normalization and plotting results, most of the Cas mutant proteins with mutations at different single amino acid sites exhibit a significantly lower editing activity than the WT Cas12j19 protein. However, Cas mutant proteins S3R, Y4R, A154R, S228R, A231R, L99R, S400R, T411R, T647R, D649R, and D658R demonstrate a 1.3 times or more higher editing efficiency than the WT Cas12j19 protein. In the Cas mutant proteins S3R, Y4R, A154R, S228R, A231R, L99R, S400R, T411R, T647R, D649R, and D658R, a 3rd amino acid, a 4th amino acid, a 154th amino acid, a 228th amino acid, a 231st amino acid, a 99th amino acid, a 400th amino acid, a 411th amino acid, a 647th amino acid, a 649th amino acid, and a 658th amino acid counting from an N terminus of the sequence shown in SEQ ID No. 1 are mutated to R, respectively.

It can be seen that the 3rd amino acid, the 4th amino acid, the 154th amino acid, the 228th amino acid, the 231st amino acid, the 99th amino acid, the 400th amino acid, the 411th amino acid, the 647th amino acid, the 649th amino acid, or the 658th amino acid counting from the N-terminus of the sequence shown in SEQ ID No. 1 is a key site for the activity of Cas12j19. Mutations at these amino acid sites can significantly enhance the editing efficiency of Cas12j19.

### Example 3 Validation of editing activities of multi-mutant Cas proteins

To further optimize the editing efficiency of Cas12j19, amino acid sites capable of enhancing the editing efficiency identified in Example 2 were combined with the mutation of a 100th amino acid in SEQ ID No. 1 to K. Activities of resulting Cas mutant proteins were validated. A target site FUT8-3: ATGGAGGCTGTCTACAATGGGGA on a GFFP sequence was selected for testing, where an underlined part was a PAM sequence. A DR sequence was GUGCUGCUGUCUCCCAGACGGGAGGCAGAACUGCAC. Acquisition and validation methods for the Cas mutant proteins were the same as those in Examples 1 to 2. Double-mutant Cas proteins E100K-S3R, E100K-L99R, E100K-A154R, E100K-A231R, E100K-S400R, E100K-T411R, E100K-T647R, E100K-D649R, and E100K-D658R were obtained.

Results are shown in FIG. 2. An editing efficiency of the WT Cas12j19 protein (WT, as shown in SEQ ID No. 1) is approximately 30%. The double-mutant Cas proteins demonstrate a significantly-improved editing efficiency, with an approximately 1-fold to 3-fold increase. The E100K-S400R double-mutant protein exhibits the optimal editing efficiency of about 90%.

The E100K-S400R double-mutant protein was further optimized. Give a too-high fluorescent mutant efficiency for FUT8-3, a FUT8-8: ATGGAAGGCTGCTTCTGTTCCCA sequence was selected as a target site for testing, where an underlined part was a PAM sequence. Construction and editing efficiency validation methods for resulting mutant proteins were the same as those in the aforementioned examples.

Results are shown in FIG. 3. When a target site is FUT8-8, an editing efficiency of the WT Cas12j19 protein (WT, as shown in SEQ ID No. 1) is 6%. Editing efficiencies of a triple-mutant protein E100K-S400R-L763R (relative to SEQ ID No. 1, a 100th amino acid is mutated to K, a 400th amino acid is mutated to R, and a 763rd amino acid is mutated to R) and a quadruple-mutant protein E100K-S400R-L763R-S45T (relative to SEQ ID No. 1, a 100th amino acid is mutated to K, a 400th amino acid is mutated to R, a 763rd amino acid is mutated to R, and a 45th amino acid is mutated to T) both are approximately 90%, which are significantly improved compared to the editing efficiency of the WT Cas12j19 protein.

The recognition of the quadruple-mutant protein E100K-S400R-L763R-S45T for different PAM sequences was tested. Results are shown in FIG. 4. The WT Cas12j19 protein exhibits significant recognition preferences for PAMs ATA and ATG. The quadruple-mutant protein E100K-S400R-L763R-S45T exhibits significant recognition preferences for all tested PAMs. Compared to the WT Cas12j19 protein, the quadruple-mutant protein E100K-S400R-L763R-S45T demonstrates a significantly improved editing efficiency for all target sites with different PAMs. The quadruple-mutant protein E100K-S400R-L763R-S45T possesses a broader PAM recognition range and a more promising application prospect than the WT Cas12j protein.

### Example 4 Validation of editing efficiencies of Cas proteins in plants

In this example, gene-editing efficiencies of the WT Cas 12j 19 protein (WT, as shown in SEQ ID No. 1), the single-mutant protein E100K (relative to SEQ ID No. 1, a 100th amino acid is mutated to K), and the quadruple-mutant protein E100K-S400R-L763R-S45T (relative to SEQ ID No. 1, a 100th amino acid is mutated to K, a 400th amino acid is mutated to R, a 763rd amino acid is mutated to R, and a 45th amino acid is mutated to T) were validated in rice and soybean. Gene-editing vectors were constructed using conventional techniques in the art, and genetically transformed into the rice and soybean. Primers were designed flanking a gRNA target site. Amplified fragments were subjected to Sanger sequencing to determine editing types. Editing efficiencies were calculated.

Editing efficiencies of the different Cas12j19 proteins for different target sites in the rice and soybean are shown in the following table.

| gRNA | Guide sequence of gRNA | Species-target gene | Editing efficiency (%) | | |
|---|---|---|---|---|---|
| | | | WT Cas12j19 | Single-mutant protein E100K | Quadruple-mutant protein E100K-S400R-L763R-S45T |
| 1 | ctttccacaggctttcttga | Rice-mir396 | 0 | 1.67 | 27.78 |
| 2 | gccgacatccaacctacagc | Rice-NAL | 1.72 | 16.67 | 58.33 |
| 3 | ccaacagtgccacgccgtac | Rice-Nramp | 32.76 | 63.33 | 83.33 |
| 4 | gtgaccacactcgtgagcaa | Soybean-GmFAD2-1A | 0 | 8.33 | 42.86 |
| 5 | aagtgaggagggagcgctga | Soybean-GmFAD2-1A | 0 | 33.33 | 66.67 |
| 6 | gtgaccacactcgtgagcaa | Soybean-GmFAD2-1B | 0 | 5.56 | 30.56 |
| 7 | aagtgaggagggaacgctga | Soybean-GmFAD2-1B | 0 | 19.44 | 52.78 |
| 8 | ccttcagcaagtaccaatgg | Soybean-GmFAD2-1A | 0 | 0.00 | 5.71 |
| 9 | tgtctcttcccatggacaca | Soybean-GmBADH2 | 0 | 0.00 | 22.22 |
| 10 | tgtccatgggaagagacaca | Soybean-GmBADH2 | 0 | 8.57 | 30.56 |
| 11 | ctcctgtatcccttcctatg | Soybean-GmBADH1 | 0 | 2.78 | 27.78 |

As shown in the table above, compared to the WT Cas12j19) protein and the single-mutant protein E100K, the quadruple-mutant protein E100K-S400R-L763R-S45T exhibits significantly improved editing efficiencies for tested target sites in both rice and soybean. Moreover, at some target sites for which the WT Cas12j19 protein and the single-mutant protein E100K exhibit no editing efficiency, the quadruple-mutant protein E100K-S400R-L763R-S45T still demonstrates a favorable editing efficiency.

Although the specific implementations of the present disclosure have been described in detail, those skilled in the art will understand that various modifications and changes can be made to the details according to all teachings published, and such modifications and changes are all within the protection scope of the present disclosure. The full content of the present disclosure is defined by the appended claims and any equivalents thereof.

## Claims

1. A clustered regularly interspaced short palindromic repeats (CRISPR)-associated (Cas) mutant protein, wherein corresponding to the amino acid sequence shown in SEQ ID No. 1, the mutant protein comprises a mutation of any one or more (comprising any 2, 3, 4, 5, 6, 7, 8, 9, 10, or 11) of a 400th amino acid, a 3rd amino acid, a 4th amino acid, a 99th amino acid, a 154th amino acid, a 228th amino acid, a 231st amino acid, a 411th amino acid, a 647th amino acid, a 649th amino acid, or a 658th amino acid relative to an amino acid sequence of a parental Cas protein;
preferably, corresponding to the amino acid sequence shown in SEQ ID No. 1, the Cas mutant protein further comprises a mutation of a 100th amino acid relative to the amino acid sequence of the parental Cas protein; and
more preferably, corresponding to the amino acid sequence shown in SEQ ID No. 1, the Cas mutant protein comprises simultaneous mutations of the 400th amino acid, the 100th amino acid, and a 763rd amino acid or simultaneous mutations of the 400th amino acid, the 100th amino acid, the 763rd amino acid, and a 45th amino acid relative to the amino acid sequence of the parental Cas protein.

2. A fusion protein, comprising the Cas mutant protein according to claim 1 and other modification moiety, wherein preferably, the modification moiety is selected from another protein or polypeptide, a detectable marker, or any combination thereof.

3. An isolated polynucleotide, wherein the polynucleotide is a polynucleotide sequence encoding the Cas mutant protein according to claim 1 or a polynucleotide sequence encoding the fusion protein according to claim 2.

4. A vector, comprising the polynucleotide according to claim 3 and a regulatory element operably linked thereto.

5. A CRISPR-Cas system, comprising the Cas mutant protein according to claim 1 and at least one guide RNA (gRNA),
wherein the gRNA is capable of binding to the Cas mutant protein according to claim 1.

6. A composition, comprising:
(i) a protein component selected from the Cas mutant protein according to claim 1 or the fusion protein according to claim 2; and
(ii) a nucleic acid component, which is a gRNA capable of binding to the Cas mutant protein according to claim 1,
wherein the protein component is capable of binding to the nucleic acid component to produce a complex.

7. An engineered host cell, comprising the Cas mutant protein according to claim 1 or a fusion protein according to claim 2 or a polynucleotide according to claim 3 or a vector according to claim 4 or a CRISPR-Cas system according to claim 5 or a composition according to claim 6.

8. A use of the Cas mutant protein according to claim 1 or a fusion protein according to claim 2 or a polynucleotide according to claim 3 or a vector according to claim 4 or a CRISPR-Cas system according to claim 5 or a composition according to claim 6 or a host cell according to claim 7 in any one or more of the following:
gene editing, gene targeting, gene cleavage, targeting and/or editing of a target nucleic acid, cleavage of a double-stranded DNA, a single-stranded DNA, or a single-stranded RNA, non-specific cleavage and/or degradation of a branched nucleic acid, non-specific cleavage of a single-stranded nucleic acid, nucleic acid detection, specific editing of a double-stranded nucleic acid, base editing of the double-stranded nucleic acid, or base editing of the single-stranded nucleic acid; or
in preparation of a reagent or kit for the gene editing or genome editing, detection and/or diagnosis of the target nucleic acid, modification of an organism by editing a target sequence in a target locus, treatment of a disease, targeting of a target gene, or cleavage of a target gene.

9. A method for editing a target nucleic acid, targeting the target nucleic acid, or cleaving the target nucleic acid, comprising a step of allowing the target nucleic acid to be in contact with the Cas mutant protein according to claim 1 or a fusion protein according to claim 2 or a polynucleotide according to claim 3 or a vector according to claim 4 or a CRISPR-Cas system according to claim 5 or a composition according to claim 6 or a host cell according to claim 7.

10. A kit for gene editing, gene targeting, or gene cleavage, comprising the Cas mutant protein according to claim 1 or a fusion protein according to claim 2 or a polynucleotide according to claim 3 or a vector according to claim 4 or a CRISPR-Cas system according to claim 5 or a composition according to claim 6 or a host cell according to claim 7.
